(19) Europäisches Patentamt
European Patent Office
Office européen des brevets

(11) Veröffentlichungsnummer: 0 306 846 A2

(12) # EUROPÄISCHE PATENTANMELDUNG

(21) Anmeldenummer: 88114315.0

(22) Anmeldetag: 02.09.88

(51) Int. Cl.4: A61K 45/06 , A61K 31/505 , A61K 31/50 , A61K 31/535 , A61K 31/47 , //(A61K31/505, 31:18,31:40,31:34,31:38), (A61K31/50,31:18,31:40,31:34, 31:38),(A61K31/535,31:40, 31:34,31:38),(A61K31/535, 31:40,31:34,31:38)

(30) Priorität: 11.09.87 US 96107

(43) Veröffentlichungstag der Anmeldung: 15.03.89 Patentblatt 89/11

(84) Benannte Vertragsstaaten: AT BE CH DE ES FR GB GR IT LI LU NL SE

(71) Anmelder: Dr. Karl Thomae GmbH
Postfach 1755
D-7950 Biberach 1(DE)

(72) Erfinder: Weisenberger,Johannes, Dr. Dipl.-Chem.
Haydnweg 5
D-7950 Biberach 1(DE)
Erfinder: Nickl, Josef, Dr. Dipl.- Chem.

Verstorben(DE)
Erfinder: Heckel, Armin, Dr. Dipl.- Chem.
Lamparterweg 1
D-7950 Biberach(DE)

(54) **Neue synergistische Kombination bestehend aus einem Phosphodiesterase-Hemmer und einem Thromboxan-A2-Antagonisten und deren Verwendung bzw. Herstellung.**

(57) Gegenstand der vorliegenden Erfindung ist die neue synergistische Kombination bestehend aus einem PDE-Hemmer und einem in den EP-A-0.253.321 und EP-A-0.262.395 beschriebenen Thromboxan $A_2$-Antagonisten und deren Verwendung zur Prophylaxe und Behandlung von venösen und arteriellen Thrombosen sowie ein Verfahren zu deren Herstellung.

EP 0 306 846 A2

**Neue synergistische Kombination bestehend aus einem Phosphodiesterase-Hemmer und einem Thromboxan-$A_2$-Antagonisten und deren Verwendung bzw. Herstellung**

Aus J. Clin. Invest. 75, 1591-1599 (1985) ist bekannt, daß die Kombination des Phosphodiesterase (PDE)-Hemmers Dipyridamol mit dem Thromboxan-Synthese-Hemmer Dazoxiben (4-[2-(1H-Imidazol-1-yl)-ethoxy]benzoesäure) keinen verbessernden Einfluß auf die antithrombotische Wirkung des PDE-Hemmers aufweist.

Überraschenderweise wurde nun gefunden, daß die Kombination bestehend aus einem Phosphodiesterase-Hemmer und einem in der EP-A-0.253.321 beschriebenen Thromboxan-$A_2$-Antagonisten der Formel

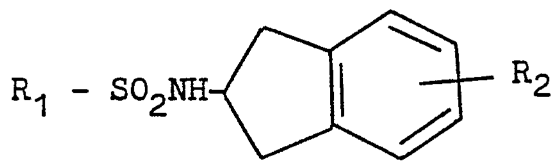

,(I)

oder einem in der EP-A-0.262.395 beschriebenen Thromboxan-$A_2$-Antagonisten der Formel

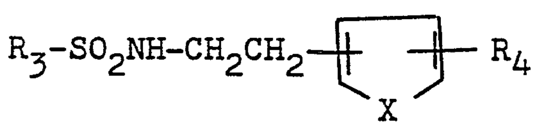

,(II)

in denen

$R_1$ eine gegebenenfalls durch ein Halogenatom, eine Methyl-oder Methoxygruppe mono- oder disubstituierte Phenylgruppe, wobei die Substituenten gleich oder verschieden sein können,

$R_2$ eine gegebenenfalls über eine geradkettige oder verzweigte Alkylengruppe mit 1 bis 5 Kohlenstoffatomen oder über eine Alkenylengruppe mit 2 bis 5 Kohlenstoffatomen gebundene Hydroxycarbonyl- oder Alkoxycarbonylgruppe mit insgesamt 2 bis 4 Kohlenstoffatomen, wobei jeweils eine Methylengruppe der vorstehend erwähnten Alkylen- oder Alkenylenreste, welche mit dem Indanylrest verknüpft sein muß, durch eine Hydroxymethylen- oder Carbonylgruppe ersetzt sein kann und gleichzeitig die Methylengruppe, über die die vorstehend erwähnte Alkoxycarbonylgruppe gebunden ist, durch eine weitere Alkoxycarbonylgruppe mit insgesamt 2 bis 4 Kohlenstoffatomen substituiert sein kann,

$R_3$ eine gegebenenfalls durch ein Halogenatom, eine Methyl- oder Methoxygruppe mono-, di- oder trisubstituierte Phenylgruppe, wobei die Substituenten gleich oder verschieden sein können, eine Benzyl-, Nitrophenyl-, Acetamidophenyl- oder Thienylgruppe,

$R_4$ eine über eine geradkettige oder verzweigte Alkylengruppe mit 1 bis 5 Kohlenstoffatomen oder über eine Alkenylengruppe mit 2 bis 5 Kohlenstoffatomen gebundene Hydroxycarbonyl- oder Alkoxycarbonylgruppe mit insgesamt 2 bis 4 Kohlenstoffatomen, wobei jeweils eine Methylengruppe der vorstehend erwähnten Alkylen- oder Alkenylenreste, welche mit dem heterocyclischen Rest verknüpft sein muß, durch eine Hydroxymethylen- oder Carbonylgruppe ersetzt sein kann, und

X eine gegebenenfalls durch eine Alkylgruppe mit 1 bis 3 Kohlenstoffatomen substituierte Iminogruppe, ein Sauerstoff- oder Schwefelatom bedeuten, und deren physiologisch verträglichen Säureadditionssalze eine viel stärkere antithrombotische Wirkung als die Einzelsubstanzen aufweist.

Gegenstand der vorliegenden Erfindung ist somit die neue synergistische Kombination bestehend aus einem PDE-Hemmer und einem der oben erwähnten Thromboxan $A_2$-Antagonisten und deren Verwendung sowie ein Verfahren zu deren Herstellung.

In der Literatur werden zahlreiche PDE-Hemmer beschrieben. Als PDE-Hemmer seien daher beispielsweise die aus der Pyrimido[5,4-d]pyrimidin-Reihe (siehe US-A-3.031.450, US-A-3.322.755 und US-A-4.518.596), aus der Isochinolinreihe (siehe US-A-3.975.524), aus der Pyrido[3,2-d]pyridiminreihe (siehe US-A-3.843.638), aus der Indolin- und Carbostyrilreihe (siehe US-A-4.329.347, GB-B-2.098.595 und US-A-4.442.111), aus der Benzoxazin-2-on-Reihe (siehe US-A-4.518.597), aus der Pyrimidinreihe (siehe US-A-3.975.384) und aus der Benzimidazolreihe (siehe US-A-4.361.563) erwähnt.

Als Einzelverbindungen seien hier beispielsweise folgende genannt:

Papaverin,
2,6-Bis(diäthanolamino)-4,8-dipiperidino-pyrimido[5,4-d]pyrimidin (Dipyridamol),
2,6-Bis(diäthanolamino)-4-piperidino-pyrimido[5,4-d]pyrimidin (Mopidamol),
6,7-Dichlor-1,5-dihydro-imidazo[2,1-b]chinazolin-2(3H)-on (Anagrelid),
2-(4-Methoxy-phenyl)-5(6)-(5-methyl-3-oxo-4,5-dihydro-2H-6-pyridazinyl)-benzimidazol (Pimobendan),
2-(4-Hydroxy-phenyl)-5(6)-(5-methyl-3-oxo-4,5-dihydro-2H-6-pyridazinyl)-benzimidazol,
6-Äthoxy-5-nitro-4-(1-oxido-thiomorpholino)-2-piperazino-pyrimidin,
7-Brom-1,5-dihydro-3,6-dimethyl-imidazo[2,1-b]chinazolin-2(3H)-on,
6-[4-(1-Cyclohexyl-1H-5-tetrazolyl)-butoxy]-2-oxo-tetrahydrochinolin,
7-Äthoxy-carbonyl-6,8-dimethyl-4-hydroxymethyl-1(2H)-phthalazinon (Phthalazinol) und
3,4-Dihydro-1-methyl-6-(1,4,5,6-tetrahydro-4-methyl-6-oxo-3-pyridazinyl)-2(1H)-chinolinon sowie deren physiologisch verträglichen Säureadditionssalze.

Die perorale Tagesdosis beträgt beispielsweise
für Papaverin 1 bis 2 mg/kg, vorzugsweise 1,5 mg/kg,
für Dipyridamol 2,5 bis 7,5 mg/kg, vorzugsweise 5 mg/kg,
für Mopidamol 15 bis 25 mg/kg, vorzugsweise 20 mg/kg,
für Anagrelid 0,05 bis 0,15 mg/kg, vorzugsweise 0,1 mg/kg,
für 2-(4-Methoxy-phenyl)-5(6)-(5-methyl-3-oxo-4,5-dihydro-2H-6-pyridazinyl)-benzimidazol 0,05 bis 0,15 mg/kg, vorzugsweise 0,08 bis 0,10 mg/kg,
für 2-(4-Hydroxy-phenyl)-5(6)-(5-methyl-3-oxo-4,5-dihydro-2H-6-pyridazinyl)-benzimidazol 0,05 bis 0,15 mg/kg, vorzugsweise 0,08 bis 0,10 mg/kg,
für 6-Äthoxy-5-nitro-4-(1-oxido-thiomorpholino)-2-piperazino-pyrimidin 0,05 bis 0,15 mg/kg, vorzugsweise 0,08 bis 0,10 mg/kg,
für 7-Brom-1,5-dihydro-3,6-dimethyl-imidazo[2,1-b]chinazolin-2-(3H)-on 0,075 bis 0,15 mg/kg, vorzugsweise 0,1 mg/kg,
für 6-[4-(1-Cyclohexyl-1H-5-tetrazolyl)-butoxy]-2-oxo-tetra-hydrochinolin 2,0 bis 4,0 mg/kg, vorzugsweise 3 mg/kg,
für Phthalazinol 2,5 bis 7,0 mg/kg, vorzugsweise 4,0 mg/kg und
für 3,4-Dihydro-1-methyl-6-(1,4,5,6-tetrahydro-4-methyl-6-oxo-3-pyridazinyl)-2(1H)-chinolinon 0,05 bis 0,15 mg/kg, vorzugsweise 0,1 mg/kg.

Als besonders geeignete PDE-Hemmer haben sich hierbei erfindungsgemäß die PDE-Hemmer aus der Benzimidazolreihe wie Pimobendan oder aus der Pyrimido[5,4-d]pyrimidin-Reihe wie Dipyridamol oder Mopidamol erwiesen.

Als Thromboxan $A_2$-Antagonisten seien beispielsweise folgende Verbindungen genannt:
4-[2-Benzolsulfonamido-indanyl-(5)]-4-oxo-buttersäure-methyl-ester,
4-[2-Benzolsulfonamido-indanyl-(5)]-4-oxo-buttersäure,
4-[2-p-Fluorbenzolsulfonamido-indanyl-(5)]-4-oxo-buttersäure-methylester,
4-[2-p-Fluorbenzolsulfonamido-indanyl-(5)]-4-oxo-buttersäure,
4-[2-p-Toluolsulfonamido-indanyl-(5)]-4-oxo-buttersäure-methyl-ester,
4-[2-p-Toluolsulfonamido-indanyl-(5)]-4-oxo-buttersäure,
4-[2-o-Methoxybenzolsulfonamido-indanyl-(5)]-4-oxo-buttersäure-methylester,
4-[2-o-Methoxybenzolsulfonamido-indanyl-(5)]-4-oxo-buttersäure,
4-[2-p-Methoxybenzolsulfonamido-indanyl-(5)]-4-oxo-buttersäure-methylester,
4-[2-p-Methoxybenzolsulfonamido-indanyl-(5)]-4-oxo-buttersäure,
3-Methyl-4-[2-benzolsulfonamido-indanyl-(5)]-4-oxo-buttersäure-methylester,
3-Methyl-4-[2-benzolsulfonamido-indanyl-(5)]-4-oxo-buttersäure,
3-Methyl-4-[2-p-fluorbenzolsulfonamido-indanyl-(5)]-4-oxobuttersäure-methylester,
3-Methyl-4-[2-p-fluorbenzolsulfonamido-indanyl-(5)]-4-oxobuttersäure,
3-Methyl-4-[2-p-toluolsulfonamido-indanyl-(5)]-4-oxo-buttersäure-methylester,
3-Methyl-4-[2-p-toluolsulfonamido-indanyl-(5)]-4-oxo-buttersäure,
4-[2-p-Chlorbenzolsulfonamido-indanyl-(5)]-4-oxo-buttersäure,
4-[2-o-Toluolsulfonamido-indanyl-(5)]-4-oxo-buttersäure,
4-[2-o-Toluolsulfonamido-indanyl-(5)]-4-oxo-buttersäure-methylester,
[2-Benzolsulfonamido-indanyl-(5)]-carbonsäure,
[2-Benzolsulfonamido-indanyl-(5)]-essigsäure,
3-[2-Benzolsulfonamido-indanyl-(5)]-crotonsäure-äthylester,
3-[2-Benzolsulfonamido-indanyl-(5)]-crotonsäure,
3-[2-Benzolsulfonamido-indanyl-(5)]-buttersäure-methylester,

3-[2-Benzolsulfonamido-indanyl-(5)]-buttersäure,
4-[2-Benzolsulfonamido-indanyl-(5)]-buttersäure-methylester,
4-[2-Benzolsulfonamido-indanyl-(5)]-buttersäure,
4-[2-p-Chlorbenzolsulfonamido-indanyl-(5)]-buttersäure-methylester,
4-[2-p-Chlorbenzolsulfonamido-indanyl-(5)]-buttersäure,
4-[2-p-Toluolsulfonamido-indanyl-(5)]-buttersäuremethylester,
4-[2-p-Toluolsulfonamido-indanyl-(5)]-buttersäure,
4-[2-o-Toluolsulfonamido-indanyl-(5)]-buttersäuremethylester,
4-[2-p-Toluolsulfonamido-indanyl-(5)]-buttersäure,
4-Hydroxy-4-[2-p-toluolsulfonamid-indanyl-(5)]-buttersäure,
[2-p-Chlorbenzolsulfonamido-indanyl-(5)]-essigsäuremethyl-ester,
[2-p-Chlorbenzolsulfonamido-indanyl-(5)]-essigsäure,
[2-p-Toluolsulfonamido-indanyl-(5)]-essigsäure-methylester,
[2-p-Toluolsulfonamido-indanyl-(5)]-essigsäure,
[2-o-Toluolsulfonamido-indanyl-(5)]-essigsäure-methylester,
[2-o-Toluolsulfonamido-indanyl-(5)]-essigsäure,
[2-p-Methoxybenzolsulfonamido-indanyl-(5)]-essigsäure-methylester,
[2-p-Methoxybenzolsulfonamido-indanyl-(5)]-essigsäure,
[2-o-Methoxybenzolsulfonamido-indanyl-(5)]-essigsäure-methylester,
[2-o-Methoxybenzolsulfonamido-indanyl-(5)]-essigsäure,
[2-(2,5-Dichlorbenzolsulfonyl)amido-indanyl-(5)]-essigsäure-methylester,
[2-(2,5-Dichlorbenzolsulfonyl)amido-indanyl-(5)]-essigsäure,
[2-p-Fluorbenzolsulfonamido-indanyl-(5)]-essigsäure-methylester,
[2-p-Fluorbenzolsulfonamido-indanyl-(5)]-essigsäure,
2-[2-p-Toluolsulfonamido-indanyl-(5)]-propionsäure-methylester,
2-[2-p-Toluolsulfonamido-indanyl-(5)]-propionsäure,
2-[2-p-Chlorbenzolsulfonamido-indanyl-(5)]-propionsäure-methylester,
2-[2-p-Chlorbenzolsulfonamido-indanyl-(5)]-propionsäure,
2-Carbäthoxy-4-[2-p-toluolsulfonamido-indanyl-(5)]-4-oxobuttersäure-äthylester,
2-Carbäthoxy-4-[2-p-chlorbenzolsulfonamido-indanyl-(5)]-oxobuttersäure-äthylester,
4-[2-(2-(p-Chlorbenzolsulfonamido)-äthyl)-1-methylpyrrol-5-yl]-4-oxobuttersäure-methylester,
4-[2-(2-Benzolsulfonamido-äthyl)-1-methylpyrrol-5-yl]-4-oxobuttersäure-methylester,
4-[2-(2-(p-Fluorbenzolsulfonamido)-äthyl)-1-methylpyrrol-5-yl]-4-oxobuttersäure-methylester,
4-[2-(2-Benzylsulfonamido-äthyl)-1-methylpyrrol-5-yl]-4-oxobuttersäure-methylester,
4-[2-(2-Thiophen-2-ylsulfonamido-äthyl)-1-methylpyrrol-5-yl]-4-oxobuttersäure-methylester,
4-[2-(2-(2,4,5-Trichlorbenzolsulfonamido)-äthyl)-1-methylpyrrol-5-yl]-4-oxobuttersäure-methylester,
4-[2-(2-(p-Toluolsulfonamido)-äthyl)-1-methylpyrrol-5-yl]-4-oxobuttersäure-methylester,
4-[2-(2-(p-Acetamidobenzolsulfonamido)-äthyl)-1-methyl-pyrrol-5-yl]-4-oxobuttersäure-methylester,
4-[2-(2-(p-Nitrobenzolsulfonamido)-äthyl)-1-methylpyrrol-5-yl]-4-oxobuttersäure-methylester,
4-[2-(2-(o-Methoxybenzolsulfonamido)-äthyl)-1-methylpyrrol-5-yl]-4-oxobuttersäure-methylester,
4-[2-(2-(p-Chlorbenzolsulfonamido)-äthyl)-1-methylpyrrol-5-yl]-4-oxobuttersäure,
4-[2-(2-Benzolsulfonamido-äthyl)-1-methylpyrrol-5-yl]-4-oxo-buttersäure,
4-[2-(2-(p-Fluorbenzolsulfonamido)-äthyl)-1-methylpyrrol-5-yl]-4-oxobuttersäure,
4-[2-(2-Benzylsulfonamido-äthyl)-1-methylpyrrol-5-yl]-4-oxobuttersäure,
4-[2-(2-Thiophen-2-yl-sulfonamido-äthyl)-1-methylpyrrol-5-yl]-4-oxobuttersäure,
4-[2-(2-(2,4,5-Trichlorbenzolsulfonamido)-äthyl)-1-methylpyrrol-5-yl]-4-oxobuttersäure,
4-[2-(2-(p-Toluolsulfonamido)-äthyl)-1-methylpyrrol-5-yl]-4-oxo-buttersäure,
4-[2-(2-(p-Acetamido-benzolsulfonamido)-äthyl)-1-methylpyrrol-5-yl]-4-oxobuttersäure,
4-[2-(2-(p-Nitrobenzolsulfonamido)-äthyl)-1-methylpyrrol-5-yl]-4-oxobuttersäure,
4-[2-(2-(2,5-Dichlorbenzolsulfonamido)-äthyl)-1-methylpyrrol-5-yl]-4-oxobuttersäure,
4-[2-(2-Benzolsulfonamido-äthyl)-furan-5-yl]-4-oxobuttersäure,
4-[2-(2-Benzolsulfonamido-äthyl)-thiophen-5-yl]-4-oxobuttersäure,
4-[2-(2-Benzolsulfonamido-äthyl)-thiophen-5-yl]-4-oxobuttersäure,
[2-(2-(4-Fluorbenzolsulfonamido)-ethyl)-1-methylpyrrol-5-yl]-essigsäure-äthylester,
[2-(2-(4-Chlorbenzolsulfonamido)-ethyl)-1-methylpyrrol-5-yl]-essigsäure-äthylester,
[2-(2-Toluolsulfonamido)-ethyl)-1-methylpyrrol-5-yl]-essigsäure-äthylester,
[2-(2-Benzylsulfonamido)-ethyl)-1-methylpyrrol-5-yl]-essigsäure-äthylester,
[2-(2-(4-Fluorbenzolsulfonamido)-ethyl)-1-methylpyrrol5-yl]-essigsäure,

[2-(2-(4-Chlorbenzolsulfonamido)-ethyl)-1-methylpyrrol5-yl]-essigsäure,
[2-(2-Toluolsulfonamido)-ethyl)-1-methylpyrrol-5-yl]-essigsäure und
[2-(2-Benzylsulfonamido)-ethyl)-1-methylpyrrol-5-yl]-essigsäure
sowie deren physiologisch verträgliche Additionssalze.

Als besonders geeignete Thromboxan $A_2$-Antagonisten haben sich erfindungsgemäß diejenigen Thromboxan $A_2$-Antagonisten erwiesen, in denen
$R_1$ eine gegebenenfalls durch ein Fluor- oder Chloratom, durch eine Methyl- oder Methoxygruppe substituierte Phenylgruppe,
$R_2$ eine Hydroxycarbonylmethyl-, 3-Hydroxycarbonyl-n-propyl-oder 3-Hydroxycarbonyl-n-propan-1-on-(1)-yl-gruppe,
$R_3$ eine gegebenenfalls durch eine Methyl-, Methoxy- oder Nitrogruppe substituierte Phenylgruppe, eine durch ein Fluor-, Chlor- oder Bromatom mono-, di- oder trisubstituierte Phenylgruppe,
$R_4$ eine Hydroxycarbonylmethyl-, Äthoxycarbonylmethyl-, 2-Äthoxycarbonyl-äthyl-, 3-Hydroxycarbonyl-n-propan-(1)-yl-, 3-Methoxycarbonyl-n-propan-(1)-yl-, 3-Hydroxycarbonyl-n-propan-1-on-(1)-yl- oder 3-Methoxycarbonyl-n-propan-1-on-(1)-yl-gruppe und
X eine methyliminogruppe, ein Sauerstoff- oder Schwefelatom bedeuten, und deren physiologisch verträglichen Additionssalze.

Hierbei haben sich die folgenden Verbindungen als besonders wertvoll erwiesen:
4-[2-p-Chlorbenzolsulfonamido-indanyl-(5)]-4-oxo-buttersäure,
4-[2-p-Toluolsulfonamido-indanyl-(5)]-4-oxo-buttersäure,
[2-p-Chlorbenzolsulfonamido-indanyl-(5)]-essigsäure,
4-[2-p-Chlorbenzolsulfonamido-indanyl-(5)]-buttersäure,
4-[2-(2-Benzolsulfonamido-äthyl)-1-methylpyrrol-5-yl]-4-oxobuttersäure,
4-[2-(2-(p-Chlorbenzolsulfonamido)-äthyl)-1-methyl-pyrrol-5-yl]-4-oxobuttersäure,
4-[2-(2-Thiophen-2-yl-sulfonamido-äthyl)-1-methyl-pyrrol-5-yl]-4-oxobuttersäure und
4-[2-(2-(p-Fluorbenzolsulfonamido)-äthyl)-1-methyl-pyrrol-5yl]-4-oxobuttersäure
sowie deren physiologisch verträglichen Additionssalze.

Beispielsweise beträgt die perorale Tagesdosis für die vorstehend genannten Verbindungen 5,0 bis 20 mg/kg, vorzugsweise 7,5 bis 12 mg/kg.

Die synergistische Wirkung der neuen Kombination wurde beispielsweise an der Kombination 6-Äthoxy-5-nitro-4-(1-oxidothiomorpholino)-2-piperazino-pyrimidin (Substanz A)/ 4-[2-p-Chlorbenzolsulfonamido-indan-5-yl]-buttersäure (Substanz B) im Vergleich zu den Einzelsubstanzen wie folgt geprüft:

Ratten mit einem durchschnittlichen Körpergewicht von 250 bis 300 g wurden mit 60 mg/kg Pentobarbital narkotisiert. Aus der abdominalen Arterie wurde 4,5 ml Blut entnommen und in 0,5 ml Zitrat (3,8 %) gegeben. Das Blut von 3 Ratten wurde jeweils gepoolt und Proben hiervon, die Lösungsmittel, die Einzelsubstanzen oder die zu untersuchende Kombination enthielten, wurden 10 Minuten lang rotiert. Von diesen Proben wurden jeweils 200 µl in Aggregometer-Küvetten gegeben und 5 Minuten lang bei 37°C gerührt. Anschließend wurden 10 µl Collagenlösung (Endkonzentration: 7 µg/ml) zugegeben und zu verschiedenen Zeiten jeweils 10 µl Blut entnommen, welches anschließend mit Formaldehyd fixiert wurde. In diesen Proben wurden die nicht aggregierten Plättchen mit einem Ultra-Flo 100 (Becton-Dickinson) bestimmt. Die nachfolgende Tabelle enthält die gefundenen Werte:

| Substanz | Konzentration | % Hemmung |
|---|---|---|
| A | 0,03 µMol/l | 4 |
| B | 0,10 µMol/l | 3 |
| A + B | | 23 |
| A | 0,03 µMol/l | 4 |
| B | 0,30 µMol/l | 19 |
| A + B | | 32 |

Desweiteren wurde die synergistische Wirkung auf die Plättchenaggregation im Gesamtblut für die Kombination, bestehen aus Pimobendan (Substanz C) und Substanz B in Vergleich zu den Einzelverbindungen wie folgt untersucht:

Ratten mit einem durchschnittlichen Körpergewicht von 250 bis 300 g Körpergewicht wurden 5 mg/kg

Substanz C und 0,5 mg/kg Substanz B oder die Kombination im Vergleich zu Kontrollen, welche nur das Vehikel appliziert bekamen, gegeben. Im Blut dieser Tiere wurde die collageninduzierte Aggregation, wie oben beschrieben, bestimmt. Die nachfolgende Tabelle enthält die gefundenen Werte:

| Substanz | Dosis | % Hemmung |
|---|---|---|
| C | 5,0 mg/kg | -1 |
| B | 0,5 mg/kg | 59 |
| B + C | | 95 |

Die an der Ratte geprüfte Kombination, nämlich 5 mg/kg Substanz C + 0,5 mg/kg Substanz B, ist nicht toxisch, da bei ihrer Applikation keine toxischen Nebenwirkungen beobachtet werden konnten.

Auf Grund ihrer pharmakologischen Eigenschaften eignet sich die neue Kombination zur Behandlung und Prophylaxe von venösen und arteriellen Thrombosen, also zur Verhütung oder Behandlung thromboembolischer Ereignisse, die mit einem pathologischen Thrombozytenverhalten einhergehen, wie z.B. nach Herzklappen- oder Gefäßoperationen, bei tiefen Beinvenenthrombosen, bei thrombotisch-thrombopenischen Purpura, nach Coronarinfarkt, nach Cerebralinfarkt, bei sogn. transient ischaemic attacks, bei Amaurosis fugax und zur Prophylaxe der Arteriosklerose.

Die Einzeldosis der neuen Kombination setzt sich hierbei zweckmäßigerweise aus der jeweils am Menschen gebräuchlichen Einzeldosis des verwendeten PDE-Hemmers und des Thromboxan $A_2$-Antagonisten zusammen. Hierbei kann insbesondere auf Grund der guten Verträglichkeit die Dosis des Thromboxan $A_2$-Antagonisten in einem großen Dosisbereich, beispielsweise zwischen 10 und 1000 mg, variiert werden.

Am Beispiel der vorstehend geprüften Kombination bedeutet dies, daß die Einzeldosis dieser Kombination 50 - 100 mg Substanz C, vorzugsweise jedoch 75 mg Substanz C, plus 10-200 mg Substanz B, vorzugsweise 10 bis 100 mg Substanz B, bei einer 3- bis 4- mal täglichen Applikation enthält.

Zur pharmazeutischen Anwendung läßt sich die neue Kombination, enthaltend 1 bis 500 mg eines PDE-Hemmers, vorzugsweise jedoch 2 bis 75 mg, plus 10 bis 300 mg eines Thromboxan/$A_2$-Antagonisten, vorzugsweise jedoch 10 bis 200 mg, sowie deren physiologisch verträgliche Additionssalze zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Was ser/Polyäthylenglykol, Propylenglykol, Cetylstearylalkohol, Carboxymethylcellulose oder fetthaltige Substanzen wie Hartfett oder deren geeignete Gemische, in übliche galenische Zubereitungen wie Tabletten, Dragées, Kapseln, Pulver, Suspensionen oder Zäpfchen einarbeiten.

Zur Erzielung einer entsprechen Wirkung am Erwachsenen erfolgt die Applikation der vorstehend erwähnten Einzeldosis 2 bis 4 mal täglich, vorzugsweise jedoch 3 bis 4 mal täglich.

Die nachfolgenden Beispiele sollen die Erfindung näher erläutern:

Beispiel 1

Filmtabletten, enthaltend 75 mg PDE-Hemmer + 75 mg Thromboxan $A_2$-Antagonist

Eine Pulvermischung aus
Dipyridamol 25 %
4-[2-p-Chlorbenzolsulfonamido-indan-5-yl]-buttersäure 25 %
Fumarsäure 15 %
Cellulose 20 %
Maisstärke 8 %
Polyvinylpyrrolidon 6 %
wird in einmem Mischgerät mit Wasser befeuchtet und durch ein Sieb mit der Maschenweite 1.5 mm granuliert. Nach Trocknung und erneuter Siebung mischt man 1 % Magnesiumstearat zu und stellt 10 mm bikonvexe Tabletten von 300 mg her. Diese Tabletten werden solange mit Hydroxypropylmethylcelluloselack besprüht bis sie 312 mg wiegen.

Beispiel 2

Hartgelatinekapseln, enthaltend 50 mg PDE-Hemmer + 200 mg Thromboxan $A_2$-Antagonist

10 kg Dipyridamol, 20 kg Fumarsäure, 11,5 kg Polyvinylpyrrolidon, 40 kg 4-[2-(2-(p-Fluorbenzolsulfonamido)-äthyl]-1-methyl-pyrrol-05-yl]-4-oxo-buttersäure, 1,5 kg Siliciumdioxid und 0,8 kg Magnesiumstearat mischt man 15 Minuten in einem Kubusmischer. Diese Mischung gibt man über einen Walzenkompaktor, dem ein Trockengranuliergerät mit Siebeinrichtung nachgeschaltet ist. Verwendung findet die Fraktion 0.25-1,0 mm. Die Kapselfüllmaschine wird so eingestellt, daß jede Kapsel der Größe 0 die 50 mg PDE-Hemmer und 200 mg Thromboxan $A_2$-Antagonist entsprechende Menge Granulat enthält.

Beispiel 3

Hartgelatinekapseln enthaltend 250 mg PDE-Hemmer + 100 mg Thromboxan $A_2$-Antagonist

a) Granulat

125 kg Mopidamol, 50 kg Fumarsäure, 13,5 kg Milchzucker werden gemischt und mit einer Lösung aus Wasser/Polyäthylenglykol 6000 befeuchtet. Nach Granulierung durch ein Sieb mit der Maschenweite 1,0 mm und Trocknung bei 45° C mischt man 1,4 kg Stearinsäure zu.

b) Dragée

100 kg 4-[2-(2-(p-Fluorbenzolsulfonamido)-äthyl]-1-methylpyrrol-5-yl]-4-oxo-buttersäure, 7,5 kg Hydroxypropylmethylcellulose, 2,5 kg Siliciumdioxid und 15 kg Carboxymethylcellulose werden mit Äthanol befeuchtet und durch ein Sieb mit der Maschenweite 1,5 mm granuliert. Nach Trocknung mischt man 1 kg Magnesiumstearat zu und verpreßt das Granulat zu 126 mg schweren bikonvexen Tabletten mit einem Durchmesser von 5,5 mm.

Diese Kerne überzieht man in mehreren Schritten mit einer Dragiersuspension, bestehend aus 5,6 kg Saccharose, 0,5 kg Gummi-arabicum und 3,8 kg Talcum, bis die Tabletten ein Gewicht von 135 mg haben.

c) Abfüllung

Auf einer Spezial-Kapselmaschine füllt man in eine Hartgelatine-Kapsel der Größe 0 long die 250 mg PDE-Hemmer ent sprechende Menge Granulat ein und legt das 100 mg Thromboxan $A_2$-Antagonist enthaltende Dragée obenauf.

Beispiel 4

Suspension, enthaltend 100 mg Dipyridamol + 10 mg Thromboxan $A_2$-Antagonist pro 5 g.

Die Suspension hat folgende Zusammensetzung:

(1) Dipyridamol 2,0 %
(2) 4-[2-p-Chlorbenzolsulfonamido-indan-5-yl]-buttersäure 0,2 %
(3) Sorbit 20,8 %
(4) Cellulose 7,5 %

(5) Natriumcarboxymethylcellulose 2,5 %
(6) Geschmackskorrigentien/Konservierungsstoffe 1,8 %
(7) Wasser 65,2 %

In heißes Wasser wird unter hoher Scherung (3) - (6) eingerührt. Nach Abkühlung werden in die viskose Suspension (1), (2) und (7) eingearbeitet.

5 g dieser Suspension enthalten 100 mg PDE-Hemmer und 10 mg Thromboxan $A_2$-Antagonist.

Beispiel 5

Depot-Form, enthaltend 200 mg Dipyridamol und 50 mg Thromboxan $A_2$-Antagonist.

a) Pellet I

Eine Mischung aus
4-[2-p-Chlorbenzolsulfonamido-indan-5-yl]-buttersäure 50,0 kg
Lysin 12,5 kg
Hydroxypropylcellulose, hochpolymer 52,5 kg
Triacetin 4,0 kg
Äthylcellulose 2,5 kg
Magnesiumstearat 3,5 kg
wird auf einem Spezialextruder mit Äthanol geknetet und in Form von Spaghetti (Durchmesser 1 mm) extrudiert, die in einem Sphäronizer zu Pellets gerundet werden. Danach trocknet man sie gründlich.

b) Pellet II

300 kg ausgemischte Weinsäure-Starterpellets werden in einem Spezialkessel mit einer Suspension bestehend aus Isopropanol, Dipyridamol und Polyvinylpyrrolidon solange besprüht, bis die entstehenden Wirkstoffpellets ca. 45 % Dipyridamol enthalten.

Diese pellets besprüht man mit einem Lack, der aus Methacrylsäure/Methylmethacrylat-Copolymer (Handelsnahme Eudragit S) und Hydroxypropylmethylcellulosephthalat (Handelsname HP 55) im Gewichtsverhältnis 85:15 bis 50:50 besteht. Die organischen Lacklösungen enthalten noch Weichmacher und Talkum.

Es werden zwei Pelletkomponenten mit 5 und 7 % Hüllenmittel und unterschiedlichem Verhältnis der Lackkomponenten in den genannten Grenzen gesprüht. Die beiden Komponenten werden so gemischt, daß sie nachfolgende in vitro-Freigabe ergeben:
Bedingungen (entsprechend USPXXI, Basket-Methode, 100 Umdrehungen/Min.,
1. Stunde künstlicher Magensaft, pH 1,2, 2. bis 6. Stunde künstlicher Darmsaft (Phosphatpuffer), pH 5.5):
Wirkstoff-Freigabe pro Stunde:

1. Stunde ca. 30 %
2. Stunde ca. 25 %
3. Stunde ca. 18 %
4. Stunde ca. 12 % nach der 6. Stunde mehr als 90 % Dipyridamol-Freigabe.

c) Abfüllung

Entsprechend dem Wirkstoffgehalt der Pelletkomponenten I und II und der gewünschten Dosierung werden die Pellets miteinander vermischt und auf einer Kapselmaschine in Kapseln der Größe 0 long abgefüllt. Die Kapsel enthält 200 mg PDE-Hemmer und 50 mg Thromboxan $A_2$-Antagonist in verzögert freigebender Form.

Beispiel 6

Ampullen, enthaltend 10 mg PDE-Hemmer + 5 mg Thromboxan $A_2$-Antagonist per 5 ml

Zusammensetzung:
(1) Dipyridamol 10 mg
(2) 4-[2-p-Chlorbenzolsulfonamido-indan-5-yl]-buttersäure 5 mg
(3) Propylenglykol 50 mg
(4) Polyäthylenglykol 5 mg
(5) Äthanol 10 mg
(6) Wasser für Injektionszwecke ad 5 ml
(7) Verd. HCl ad -pH 3

Die Wirkstoffe werden unter Erwärmung in der Lösung aus (3) - (7) gelöst. Nach pH-Kontrolle und Sterilfiltration füllt man in geeignete Ampullen und sterilisiert.

Beispiel 7

Suppositorien, enthaltend 100 mg PDE-Hemmer + 200 mg Thromboxan $A_2$-Antagonist

1 Suppositorium enthält:
(1) Papaverin 100 mg
(2) 4-[2-p-Chlorbenzolsulfonamido-indan-5-yl]-buttersäure 200 mg
(3) Fumarsäure 200 mg
Suppositorienmasse 1 500 mg

In die geschmolzene Masse werden bei 50 °C die mikronisierten Komponenten (1) - (3) agglomeratfrei suspendiert. Es werden Suppositorien mit 2 g Gewicht gegossen.

Selbstverständlich können auch andere PDE-Hemmer und Thromboxan $A_2$-Antagonisten in den in der Beschreibung erwähnten Dosierungen in die vorstehend beschriebenen pharmazeutischen Anwendungsbeispiele eingearbeitet werden.

**Ansprüche**

1. Synergistische Arzneimittelkombination, enthaltend einen Phosphodiesterasehemmer und einen Thromboxan $A_2$-Antagonisten der Formel

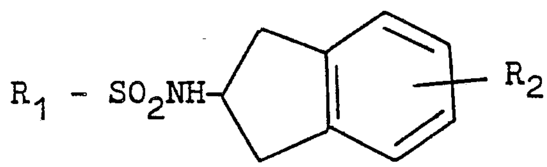
,(I)

oder der Formel

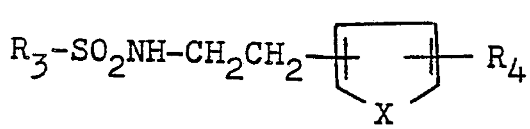
,(II)

in denen
$R_1$ eine gegebenenfalls durch ein Halogenatom, eine Methyl-oder Methoxygruppe mono- oder disubstituierte Phenylgruppe, wobei die Substituenten gleich oder verschieden sein können,
$R_2$ eine gegebenenfalls über eine geradkettige oder verzweigte Alkylengruppe mit 1 bis 5 Kohlenstoffatomen oder über eine Alkenylengruppe mit 2 bis 5 Kohlenstoffatomen gebundene Hydroxycarbonyl- oder Alkoxycarbonylgruppe mit insgesamt 2 bis 4 Kohlenstoffatomen, wobei jeweils eine Methylengruppe der vorstehend erwähnten Alkylen- oder Alkenylenreste, welche mit dem Indanylrest verknüpft sein muß, durch eine Hydroxymethylen- oder Carbonylgruppe ersetzt sein kann und gleichzeitig die Methylengruppe, über die die vorstehend erwähnte Alkoxycarbonylgruppe gebunden ist, durch eine weitere Alkoxycarbonylgruppe mit insgesamt 2 bis 4 Kohlenstoff atomen substituiert sein kann,
$R_3$ eine gegebenenfalls durch ein Halogenatom, eine Methyl-oder Methoxygruppe mono-, di- oder trisubstituierte Phenylgruppe, wobei die Substituenten gleich oder verschieden sein können, eine Benzyl-, Nitrophenyl-, Acetamidophenyl- oder Thienylgruppe,
$R_4$ eine über eine geradkettige oder verzweigte Alkylengruppe mit 1 bis 5 Kohlenstoffatomen oder über eine Alkenylengruppe mit 2 bis 5 Kohlenstoffatomen gebundene Hydroxycarbonyl- oder Alkoxycarbonylgruppe mit insgesamt 2 bis 4 Kohlenstoffatomen, wobei jeweils eine Methylengruppe der vorstehend erwähnten Alkylen- oder Alkenylenreste, welche mit dem heterocyclischen Rest verknüpft sein muß, durch eine Hydroxymethylen- oder Carbonylgruppe ersetzt sein kann, und
X eine gegebenenfalls durch eine Alkylgruppe mit 1 bis 3 Kohlenstoffatomen substituierte Iminogruppe, ein Sauerstoff-oder Schwefelatom bedeuten, und deren physiologisch verträglichen Säureadditionssalze neben einem oder mehreren inerten Trägerstoffen.

2. Arzneimittelkombination gemäß Anspruch 1, dadurch gekennzeichnet, daß diese als PDE-Hemmer einen aus der Pyrimido-[5,4-d]pyrimidin-Reihe, aus der Isochinolinreihe, aus der Pyrido[3,2-d]-pyridiminreihe, aus der Indolin- und Carbostyrilreihe, aus der Benzoxazin-2-on-Reihe, aus der Pyrimidinreihe oder aus der Benzimidazolreihe enthält.

3. Arzneimittelkombination gemäß den Ansprüchen 1 oder 2, enthaltend einen Thromboxan $A_2$-Antagonisten der Formel

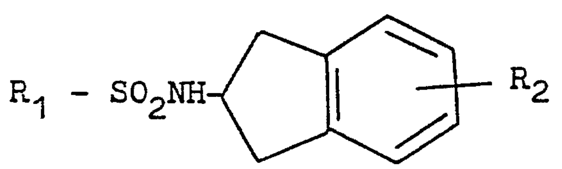

, (I)

oder der Formel

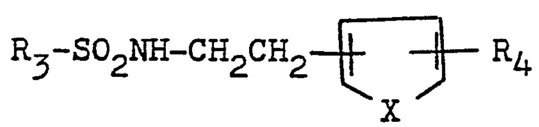

, (II)

in denen
$R_1$ eine gegebenenfalls durch ein Fluor- oder Chloratom, durch eine Methyl- oder Methoxygruppe substituierte Phenylgruppe,
$R_2$ eine Hydroxycarbonylmethyl-, 3-Hydroxycarbonyl-n-propyl- oder 3-Hydroxycarbonyl-n-propan-1-on-(1)-yl-gruppe,
$R_3$ eine gegebenenfalls durch eine Methyl-, Methoxy- oder Nitrogruppe substituierte Phenylgruppe, eine durch ein Fluor-, Chlor- oder Bromatom mono-, di- oder trisubstituierte Phenylgruppe,
$R_4$ eine Hydroxycarbonylmethyl-, Äthoxycarbonylmethyl-, 2-Äthoxycarbonyl-äthyl-, 3-Hydroxycarbonyl-n-propan-(1)-yl-, 3-Methoxycarbonyl-n-propan-(1)-yl-, 3-Hydroxycarbonyl-n-propan-1-on-(1)-yl- oder 3-Methoxycarbonyl-n-propan-1-on-(1)-yl-gruppe und
X eine Methyliminogruppe, ein Sauerstoff- oder Schwefelatom bedeuten, oder ein physiologisch verträgliches Additionssalz hiervon.

4. Arzneimittelkombination gemäß den Ansprüchen 1, 2 und 3, enthaltend einen Phosphodiesterase-Hemmer aus der Benzimidazolreihe wie Pimobendan oder aus der Pyrimido[5,4-d]-pyrimidin-Reihe wie Dipyridamol oder Mopidamol.

5. Arzneimittelkombination gemäß den Ansprüchen 1 bis 4, enthaltend den Phosphodiesterase-Hemmer Pimobendan, Dipyridamol oder Mopamidol und als Thromboxan-$A_2$-Antagonisten 4-[2-(2- (p-Fluorbenzolsulfonamido)-äthyl]-1-methyl-pyrrol-5-yl]-4-oxo-buttersäure oder 4-[2-p-Chlorbenzolsulfonamido-indan-5-yl]-buttersäure oder ein physiologisch verträgliches Additionssalz hiervon.

6. Arzneimittelkombination gemäß den Ansprüchen 1 bis 5, dadurch gekennzeichnet, daß diese 1 bis 500 mg eines PDE-Hemmers und 10 bis 300 mg eines Thromboxan $A_2$-Antagonisten enthält.

7. Verwendung einer Arzneimittelkombination gemäß den Ansprüchen 1 bis 6 zur Behandlung und Prophylaxe von venösen und arteriellen Thrombosen.

8. Verwendung einer Mischung bestehend aus einem PDE-Hemmer und einem Thromboxan $A_2$-Antagonisten gemäß den Ansprüchen 1 bis 6 zur Herstellung eines Arzneimittels, welches zur Behandlung und Prophylaxe von venösen und arteriellen Thrombosen geeignet ist.

9. Verfahren zur Herstellung eines Arzneimittels gemäß den Ansprüchen 1 bis 6, dadurch gekennzeichnet, daß auf nichtchemischem Wege ein PDE-Hemmer und ein Thromboxan $A_2$-Antagonist gemäß den Ansprüchen 1 bis 6 in einen oder mehrere inerte übliche Trägerstoffe eingearbeitet wird.